Europäisches Patentamt

European Patent Office

Office européen des brevets

(19)

(11) Publication number: **0 471 265 B1**

# EUROPEAN PATENT SPECIFICATION

(45) Date of publication of patent specification: **25.10.95**   (51) Int. Cl.⁶: **C12N 9/58**, C11D 3/386

(21) Application number: **91113109.2**

(22) Date of filing: **06.01.89**

(60) Publication number of the earlier application in accordance with Art.76 EPC: **0 394 352**

(54) **Specific protease.**

(30) Priority: **07.01.88 DK 62/88**
              **07.01.88 DK 63/88**

(43) Date of publication of application:
    **19.02.92 Bulletin 92/08**

(45) Publication of the grant of the patent:
    **25.10.95 Bulletin 95/43**

(84) Designated Contracting States:
    **AT BE CH DE FR GB IT LI LU NL SE**

(56) References cited:
    **EP-A- 0 199 404**
    **GB-A- 1 356 130**
    **US-A- 365 399**
    **US-A- 4 264 738**

(73) Proprietor: **NOVO NORDISK A/S**
    **Novo Allé**
    **DK-2880 Bagsvaerd (DK)**

(72) Inventor: **Nielsen, Ruby Ione**
    **Gedebakken 9**
    **DK-3520 Farum (DK)**
    Inventor: **Jensen, Georg Wilhelm**
    **Klirevaenget 69**
    **DK-2880 Bagsvaerd (DK)**
    Inventor: **Aaslyng, Dorrit Anita**
    **Fyrren 8,**
    **Svogerslev**
    **DK-4000 Roskilde (DK)**
    Inventor: **Schneider, Palle**
    **Rydtoften 43**
    **DK-2750 Ballerup (DK)**

(74) Representative: **Knudsen, Sten Lottrup et al**
    **c/o Novo Nordisk A/S,**
    **Patent Department,**
    **Novo Allé**
    **DK-2880 Bagsvaerd (DK)**

## Description

## TECHNICAL FIELD

This invention relates to a novel protease, which is suited for use in detergents.

## BACKGROUND ART

Proteases are widely used as ingredients in commercial detergents to improve the detergency towards proteinaceous soiling. Further information on this may be found in the article "How Enzymes got into Detergents", vol. 12, Developments in Industrial Microbiology, a publication of the Society for Industrial Microbiology, American Institute of Biological Sciences, Washington, D.C. 1971, by Claus Dambmann, Poul Holm, Villy Jensen and Mogens Hilmer Nielsen, and in P.N. Christensen, K. Thomsen and S. Branner: "Development of Detergent Enzymes", paper presented on 9 October 1986 at the 2nd World Conference on Detergents held in Montreaux, Switzerland.

As indicated in said references, trypsin preparations were previously used in detergents, but since the 1960'es alkaline Bacillus proteases have been used almost exclusively for this purpose, and in this period large efforts have been devoted to the development of microbial proteases with improved detergency. Detergents comprising alkaline Bacillus proteases are described e.g. in GB-A-1,342,784 and GB-A-1,356,130

US-A-3,652,399 discloses proteases from Fusarium sp. S-19-5 and Fusarium oxysporum f. batatas IFO 4468 and their use in detergent.

It is known that whereas pure trypsin is very specific hydrolyzing only a few peptide bonds in any given protein, the commonly used Bacillus protease have a broad specificity and thus hydrolyzing many bonds in a given substrate.

In the search for improved proteases it has been assumed that such a protease should have the broadest possible substrate specificity, i.e. it should be able to hydrolyze as many bonds as possible in the protein soiling.

Thus, M. Minagawa, Osaka Shiritsu Daigaku Seikatsu Kagaku-bu Kiyo, vol. 23, pp. 65-74 (1975) states on p. 68: "The type of protease adapted for use in the removal of protein stains must have a wide spectrum of substrate specificity capable of degrading the peptide bond, indiscriminately."

## STATEMENT OF THE INVENTION

Surprisingly, we have now discovered a novel protease from Fusarium that is amino acid-specific and shows excellent detergency.

Accordingly, the invention provides a protease, characterized by:
a) being a serine protease
b) the ability to hydrolyze the oxidized B-chain of bovine insulin, so that a chromatogram shows only two major hydrolysis products
c) showing immunochemical identity to a protease obtained by cultivation of Fusarium sp. DSM 2672
d) an isoelectric point of about 9-10
e) ability to hydrolyze Bz-Arg-pNA
f) essentially no hydrolysis of Suc-AAPF-pNA
g) essentially the same activity towards casein at pH 9 and pH 11

The invention also provides a protease preparation, a detergent additive and a detergent composition comprising the above protease.

## DETAILED EXPLANATION OF THE INVENTION

Amino acid-specificity

The protease of the present invention has trypsin-like specificity, i.e. it is an endoprotease (endopeptidase) that hydrolyzes proteins by preferentially cleaving peptide bonds adjacent to two amino acids giving rise to large peptide fragments. A number of such proteases are known, especially of animal and microbial origin. See K. Morihara: "Comparative Specificity of Microbial Proteinases", Adv. Enzymol. Relat. Areas Mol. Biol., 41, 179-243 (1974).

The protease preparation of the invention is preferably essentially devoid of other proteases. Typically, the specific protease (as defined above) makes up more than 90% and especially more than 95% of the total protease present, on weight basis or activity basis (e.g. measured in CPU, described below).

## Protease production

The novel protease was isolated from a microbial strain which has been deposited on 6 June 1983 at Deutsche Sammlung von Mikroorganismen, Göttingen, West Germany under the terms of the Budapest Treaty with the deposit No. DSM 2672. It was classified as belonging to Fusarium oxysporum.

Broth containing protease of the invention may be obtained by cultivating said strain according to principles known in the art, e.g. US-A-3,652,399 (Takeda) or to an example of this specification.

The culture broth contains at least two proteases (I and II, with II being the protease of the invention) as well as other proteins. Separation can be made by affinity chromatography on a bacitracin-sepharose column, but it was found that a column of soy bean trypsin inhibitor-sepharose (STI-sepharose) has a better capacity.

By use of 0.05M boric acid, pH 6.5 as buffer, proteases I and II will be bound, while other protein is eluted. The unwanted protease I can then be eluted by 0.25M NaCl in the same buffer, by 0.1M NaCl or by 0.05 M boric acid at pH 4-5 or below. Protease II (protease of the invention) can finally be eluted by 0.05M acetic acid, pH 2.8.

Recombinant DNA technology may be used to provide a microorganism containing a gene that encodes for and expresses and preferably also excretes the specific protease. This organism may be cultivated to produce protease for use in the invention.

### Enzyme-chemical characterization

SDS-PAGE and isoelectric focusing (IEF) in the presence of marker proteins are convenient methods for molecular weight (MW) and isoelectric point (pI) determinations, respectively. According to these methods, protease of the invention has MW of about 27 kD and pI of 9-10. Protease I (described above) has MW of about 30 kD and pI 9-10, and prior-art Fusarium protease produced by cultivation of strain S-19-5 according to US-A-3,652,399 contains a single component with MW of about 32 kD by the above method.

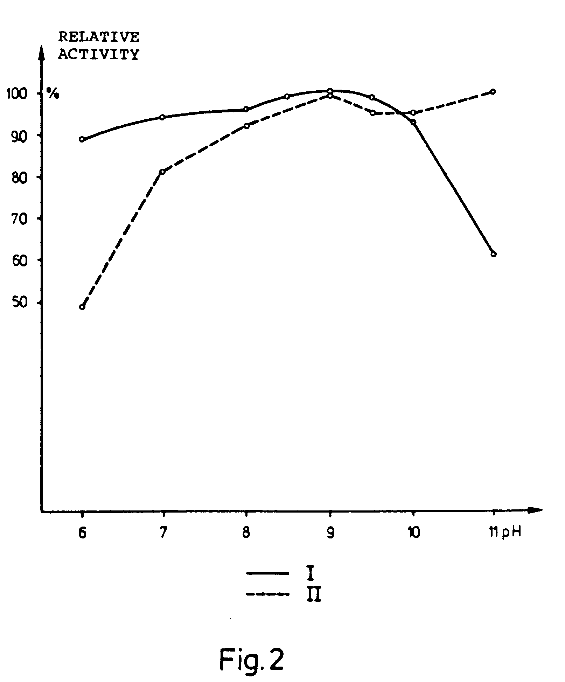

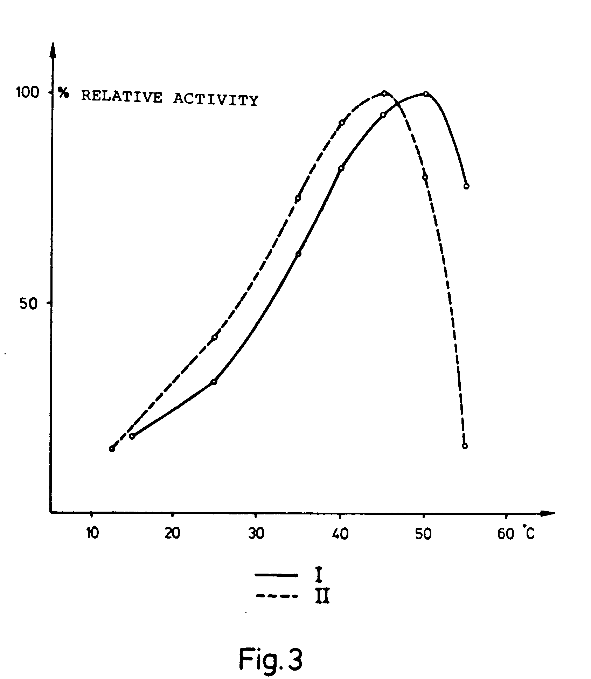

The pH and temperature dependence of activity is shown in fig. 2 and 3, where protease I is also shown for comparison. The curves are based on the CPU method (described below), except that temperature or pH is varied. As shown in the figures, protease of the invention has temperature optimum around 45°C (30 minutes reaction at pH 9.5), and pH optimum at 8.5-11.0 (30 minutes reaction at 25°C) with nearly constant activity in that pH range.

The pH and temperature curves were also measured in solutions of a built liquid detergent and of a powder detergent with perborate and TAED (bleach activator). These curves were nearly identical to those without detergent shown in figs. 2 and 3.

The protease is inhibited by inhibitors characteristic for serine proteases, such as PMSF.

To illustrate the specificity, oxidized B-chain of bovine insulin was hydrolyzed by protease (1.38 CPU/l, 15 min), and the hydrolysis products were analyzed by reverse-phase liquid chromatography (5 $\mu$m silica coated with C-18 hydrocarbon, gradient elution). Fig. 4 shows the results with protease of the invention, fig. 5 with component I from DSM 2672, and fig. 6-7 with prior-art Fusarium proteases according to US-A-3,652,399 (Fusarium sp. S-19-5 and F. oxysporum f. batatas IFO 4468, respectively).

The chromatograms show a striking difference. That of the protease of the invention has only two major peaks and appears to be similar to the chromatogram obtained with trypsin, which is known to hydrolyze two peptide bonds (Arg(22)-Gly(23) and Lys(29)-Ala(30)). In contrast, the other Fusarium proteases hydrolyze a multitude of bonds in this substrate, resulting in more than 10 peaks of comparable size.

The substrate specificity is also illustrated by the action on two synthetic substrates. Protease of the invention hydrolyzes Bz-Arg-pNA but not Suc-AAPF-pNA. Protease I, in contrast, hydrolyzes only the latter.

### Immunochemical characterization

Monospecific antisera against purified protease may be raised for example by immunizing rabbits according to the regimen described by N. Axelsen et al. in: A manual of Quantitative Immunoelectrophoresis, Blackwell Scientific Publications, 1973, chapter 23. Purified immunoglobulins may be obtained from the antisera, for example by salt precipitation (($NH_4$)$_2$$SO_4$), followed by dialysis and ion exchange chromatography, e.g. on DEAE-Sephadex.

Immunochemical characterization of proteins may be conducted either by Ouchterlony double-diffusion analysis (O. Ouchterlony in: Handbook of Experimental Immunology (D.M. Weir, Ed.), Blackwell Scientific Publications, 1967, pp. 655-706), by crossed immunoelectrophoresis (N. Axelsen et al., supra, chapters 3 and 4), or by rocket immunoelectrophoresis (N. Axelsen et al., supra, Chapter 2).

The protease of the invention shows immunochemical non-identity to protease I from DSM 2672 and to S-19-5 prior-art Fusarium protease.

Detergent additive

The detergent additive of the invention is in the form of a non-dusting granulate, a stabilized liquid or a protected enzyme.

Non-dusting granulates may be produced e.g. according to NL-C-167,993 (Novo), US-A-4,106,991 (Novo) or US-A-4,661,452 (Novo) and may optionally be coated according to principles known in the art.

A liquid protease preparation may be stabilized e.g. by adding propylene glycol, other polyols, sugars, sugar alcohols, lactic acid, boric acid. Other enzyme stabilizers are known in the art.

Protected enzyme may be produced according to EP-A-238,216 (Novo, Albright & Wilson).

The detergent additive of the invention may contain one or more other detergent enzymes, e.g. lipase, cellulase or amylase. In the case of a granulate, the enzymes may be mixed before or after granulation.

Detergent composition

The detergent compositions of the invention comprise surfactant which may be of the anionic, non-ionic, cationic, amphoteric or zwitterionic type, or a mixture of these. Typical examples of anionic surfactant are linear alkyl benzene sulfonate (LAS), alpha olefin sulfonate (AOS), alcohol ethoxy sulfate (AES) and natural soap of alkali metals.

Detergent according to the invention may contain other detergent ingredients known in the art, such as builders, bleaching agents, bleach activators, anti-corrosion agents, sequestering agents, anti-soil redeposition agents, perfumes, stabilizers for the enzymes and bleaching agents.

The detergent compositions of the invention can be formulated in any convenient form, such as powders, liquids. The protease may be stabilized in a liquid detergent by inclusion of enzyme stabilizers, e.g. those mentioned above.

Detergents usually have a pH in solution of 7-12, especially 8-10.5. Specific protease with activity at this pH is preferred.

The detergent of the invention may contain one or more other detergent enzymes in addition to protease of the invention. Examples are lipase, amylase and cellulase. It is known that when combining a protease with another enzyme in a detergent, the other enzyme becomes liable to digestion and deactivation by the protease in the detergent solution (see e.g. EP-A-205,208 (Unilever)). In this connection, the high substrate specificity of the protease of the invention makes it more compatible with other enzymes. The two (or more) enzymes may be added separately or in the form of a combined additive.

Protease activity (CPU)

Proteolytic activity is determined with casein as the substrate. One Casein Protease Unit (CPU) is defined as the amount of enzyme liberating 1 millimole of primary amino groups (determined by comparison with a serine standard) per minute under standard conditions, i.e. incubation for 30 minutes at 25°C and pH 9.5.

A 2% (w/v) solution of casein (Hammarsten, supplied by Merck A.G., West Germany) is prepared with the Universal Buffer described by Britton and Robinson (Journ.Chem.Soc. 1931, p. 1451), adjusted to pH 9.5.

Two ml of substrate solution is preincubated in a water bath for 10 minutes at 25°C. 1 ml of enzyme solution containing b g/ml of enzyme preparation, corresponding to about 0.2 - 0.3 CPU/ml of Britton-Robinson buffer (pH 9.5), is added. After 30 minutes of incubation at 25°C the reaction is terminated by the addition of a stopping agent (5 ml of a solution containing trichloroacetic acid (17.9 g), sodium acetate (29.9 g), and acetic acid (19.8 g), filled up to 500 ml with deionized water). A blank is prepared in the same manner as the test solution, except that the stopping agent is added prior to the enzyme solution.

The reaction mixtures are kept for 20 minutes in the water bath, whereupon they are filtered through Whatman® 42 paper filters.

Primary amino groups are determined by their colour development with o-phthaldialdehyde (OPA).

Disodium tetraborate decahydrate (7.62 g) and sodium dodecylsulfate (2.0 g) is dissolved in 150 ml of water. OPA (160 mg) dissolved in 4 ml of methanol is then added together with 400 $\mu$l of beta-mercaptoethanol, whereafter the solution is made up to 200 ml with water.

To the OPA reagent (3 ml) is added 400 $\mu$l of the above-mentioned filtrates with mixing. The optical density (OD) at 340 nm is measured after about 5 minutes.

The OPA test is also performed with a serine standard containing 10 mg of serine in 100 ml of Britton-Robinson buffer (pH 9.5). The buffer is used as a blank.

The protease activity is calculated from the optical density measurements by means of the following formula:

$$\text{CPU/ml of enzyme solution} = \frac{(OD_t - OD_b) \times C_{ser} \times Q}{(OD_{ser} - OD_B) \times MW_{ser} \times t_i}$$

CPU/g of enzyme preparation = CPU/ml: b

wherein $OD_t$, $OD_b$, $OD_{ser}$ an $OD_B$ is the optical density of the test solution, blank, serine standard, and buffer, respectively, $C_{ser}$ the concentration of serine in mg/ml in the standard, $MW_{ser}$ the molecular weight of serine. Q is the dilution factor (in this instance equal to 8) for the enzyme solution, and $t_i$ is the incubation time in minutes.

**BRIEF DESCRIPTION OF THE DRAWINGS**

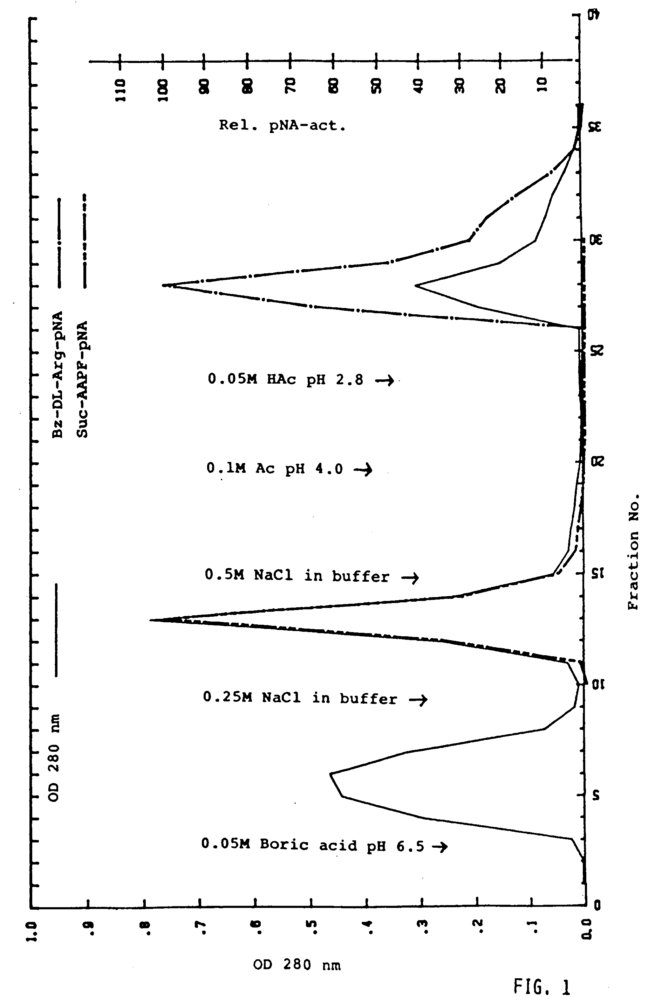

Fig. 1 shows the elution chromatogram of culture broth from strain DSM 2672 on a STI-Sepharose column. Details are given in Example 2.

Figs. 2 and 3 show the pH-activity and temperature activity curves, respectively, of the two proteases from DSM 2672, viz. protease II (protease of the invention) and protease I for comparison.

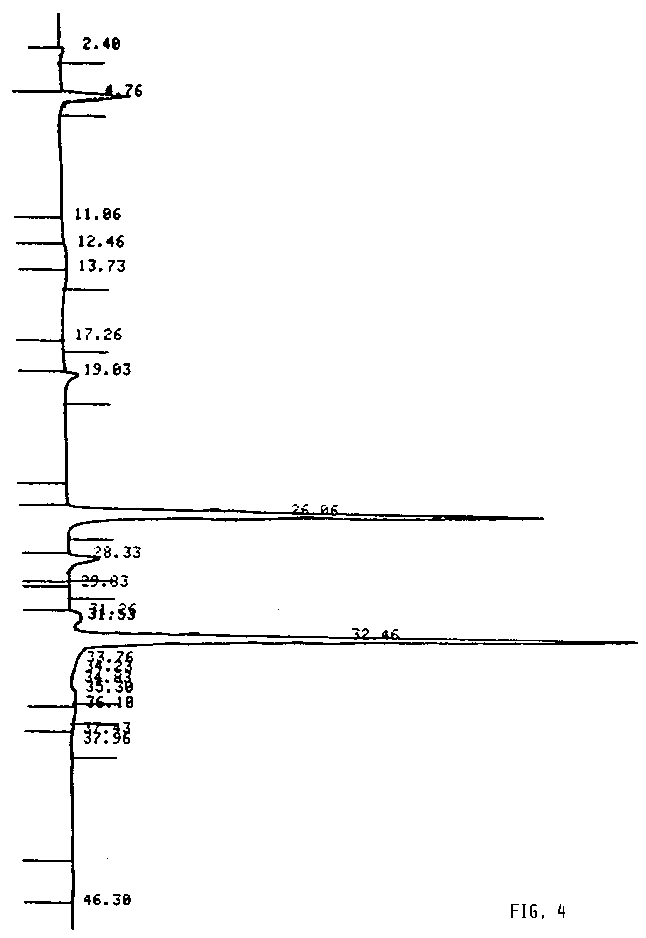

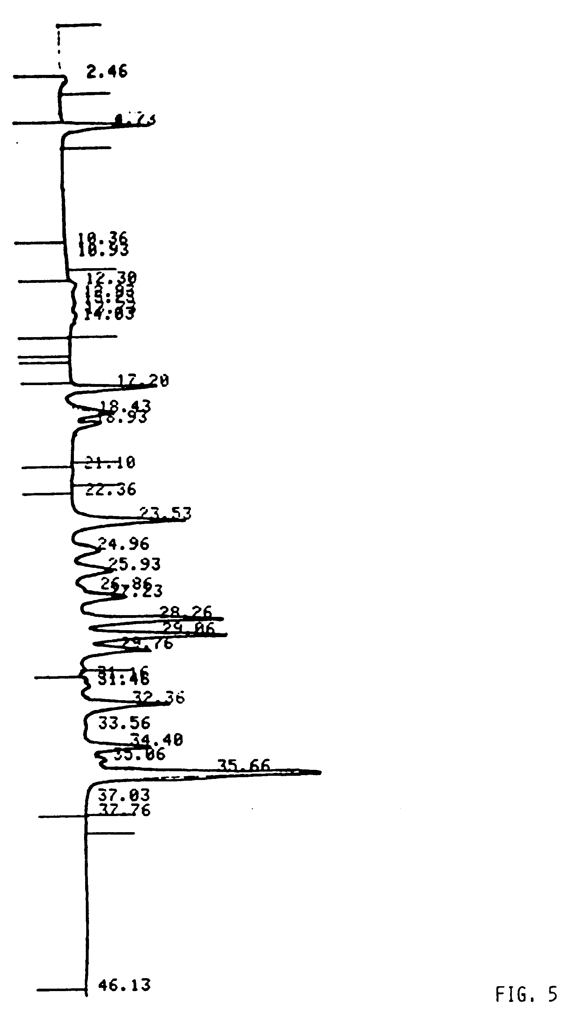

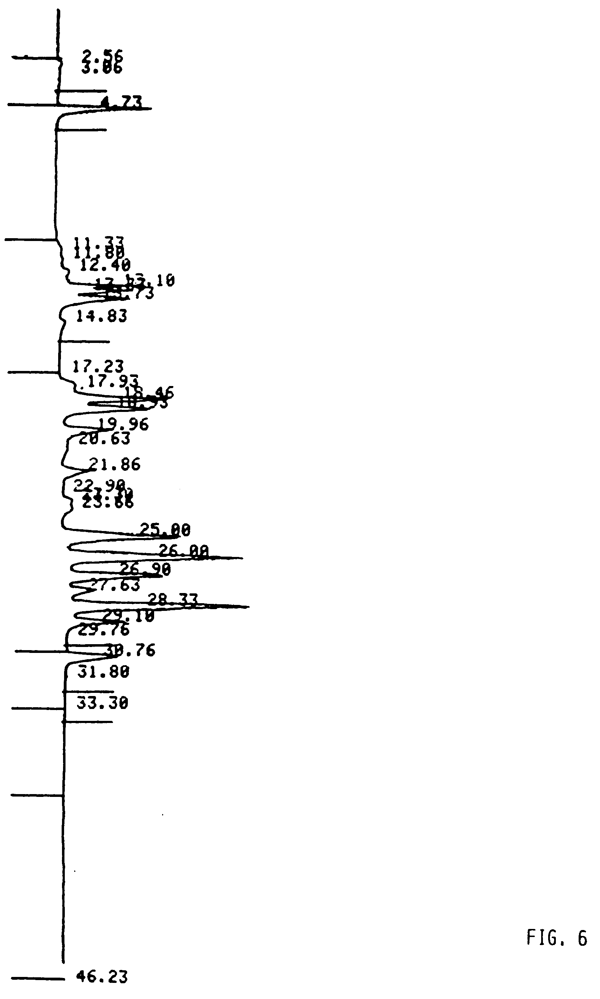

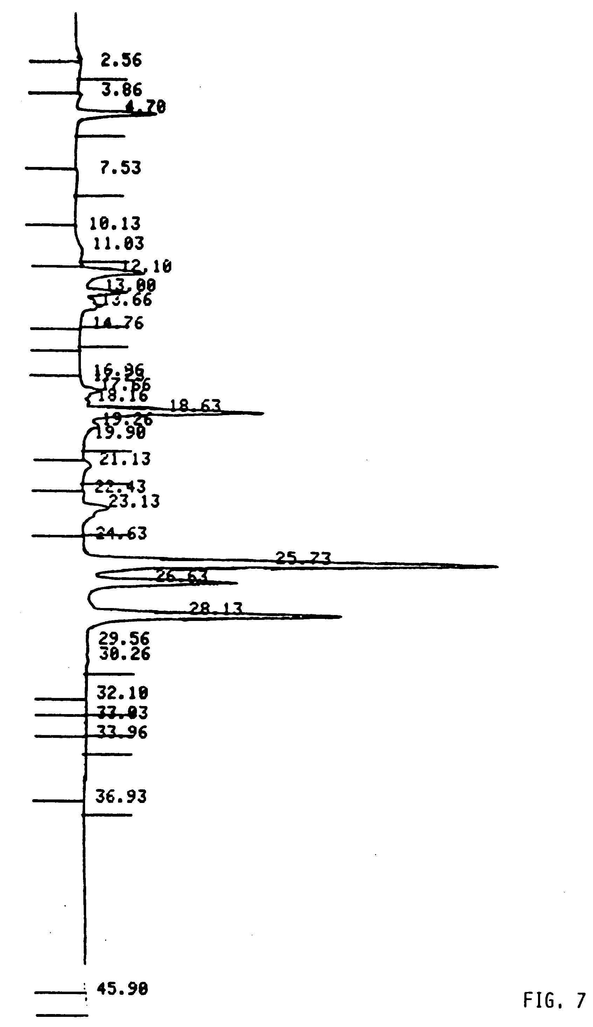

Figs. 4 - 7 show reverse-phase chromatograms of hydrolysis products of oxidized B-chain of bovine insulin with Fusarium proteases Fig. 4 shows hydrolysis with protease of the invention, fig. 5 with protease I from DSM 2672, fig. 6 with protease from S-19-5, and fig. 7 with protease from F. oxysporum f. batatas (IFO 4468), the two latter prepared according to US-A-3,652,399.

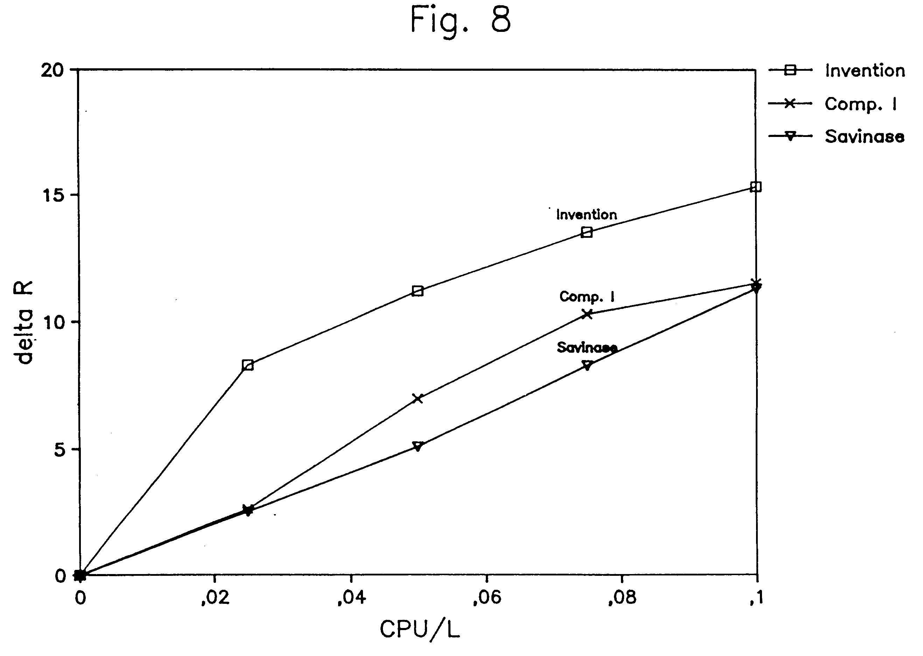

Figs. 8 - 13 show the results of the washing trials of Examples 3 - 8, respectively.

**EXAMPLES**

**EXAMPLE 1**

Fermentation of F. oxysporum DSM 2672

A seed fermenter with the below medium was inoculated with DSM 2672 and fermented for 30-35 hours with aeration. It was then used for seeding a 10 times larger main fermenter with the same medium and fermented for 114 hours with aeration and with continuous dosing of additional substrate.

Medium composition (w/v%):

| Soy meal | 5.0 |
|---|---|
| glucose | 5.0 |
| $KH_2PO_4$ | 2.0 |
| $K_2HPO_4$ | 2.0 |
| $CaCl_2.2H_2O$ | 0.02 |
| $MgSO_4.7H_2O$ | 0.02 |
| Soy bean oil | 0.5 |
| Pluronic® (ml/l) | 0.033 |

Substrate dosed: 45% w/v glucose

dosing rate: 0.28% vol/vol/hour

The protease activity of the broth was 2.84 AU/l (AU indicates protease activity in Anson Units, see US-A-3,723,250, col. 8).

Crude protease was recovered from the culture broth by addition of ammonium sulfate (salting out), filtration, redissolution of filter cake, purification by ion exhange followed by bentonite, and finally drying.

**EXAMPLE 2**

Separation of proteases

Crude protease obtained as in Example 1 (activity 15 CPU/g) was dissolved in buffer (0.05M boric acid, pH 6.5). After decolorization by adsorption on DEAE-Sephadex, the OD was 1.2 at 280 nm. 15 ml of this solution with a total protein content of 18 mg (calculated from OD, 280 nm), was separated by affinity chromatography.

The gel was soy bean trypsin inhibitor-agarose (STI-agarose), the bed volume was 10 ml, and the column diameter was 2.5 cm. The above buffer was used, with a flow rate of 0.5 ml/min, and fractions of 4.5 ml each were collected. For each fraction, OD 280 nm was measured, as well as activity towards Bz-DL-Arg-pNA and Suc-AAPF-pNA. The elution chromatogram is shown in fig. 1. The eluent used at each stage is also indicated in the figure.

As shown in fig. 1, unadsorbed material was first eluted with buffer, then a protease peak (termed I) was eluted by 0.25M NaCl in buffer. As shown, increase to 0.5M NaCl and application of 0.1M acetate, pH 4.0 eluted no further material. A second protease peak (termed II) was eluted by 0.05M acetic acid, pH 2.8.

Distribution of protein:

```
        Injected                                  18 mg
        Eluted
            Not adsorbed      7.45 mg (41%)
            Component  I      6.55 mg (36%)
            Component II      4.03 mg (22%)
            Total                                 18 mg
```

Approx. 64% of the protein remained in solution after the initial purification by DEAE, so the protein recovery in relation to the protease concentrate was as follows:

Component I 23% of protein (62% of protease)

Component II 14% of protein (38% of protease)

**EXAMPLES 3 - 8**

Washing trials

The following detergent solutions (in g/l) were used. Nos. I-III represent powder detergents, and No. IV a liquid built detergent.

|  | I | II | III |
|---|---|---|---|
| LAS | 0.4 | 0.4 | 0.4 |
| AE | 0.15 | 0.15 | 0.15 |
| Soap | 0.15 | 0.15 | 0.15 |
| Sodium tripolyphosphate | 1.75 | - | 1.75 |
| Sodium silicate | 0.4 | 0.4 | 0.4 |
| Carboxy methyl cellulose | 0.05 | 0.05 | 0.05 |
| EDTA | 0.01 | 0.01 | 0.01 |
| Sodium sulfate | 2.1 | 2.1 | 2.1 |
| Sodium perborate | - | - | 1.0 |
| TAED | - | - | 0.1 |
| Zeolite A | - | 1.25 | - |
| NTA | - | 0.5 | - |
| Sodium carbonate | - | 0.5 | - |
| NaOH to pH: | 9.5 | 10.0 | 9.5 |

|  | IV |
|---|---|
| AES | 0.23 |
| AE | 0.23 |
| Oleic acid | 0.075 |
| Triethanolamine | 0.15 |
| Ethanol | 0.03 |
| Propylene glycol | 0.15 |
| DTPA | 0.008 |
| Disodium citrate, $2H_2O$ | 0.17 |
| $CaCl_2$, $2H_2O$ | 0.015 |
| NaOH to pH: | 8.0 |

LAS is linear alkyl benzene sulfonate (Nansa® 80S, product of Albright & Wilson, UK), AE is alcohol ethoxylate (Berol® 065, product of Berol Kemi AB, Sweden), EDTA is ethylene diamine tetra-acetic acid, TAED is N,N,N,N-tetra-acetyl ethylene diamine, NTA is nitrilotriacetic acid, AES is alcohol ethoxy sulfate (Dobanol® 25-3S, product of Shell Chemicals), DTPA is diethylene triamine penta-acetic acid tri-sodium mono-calcium salt.

Soiled spinach swatches were made on a Mathis Washing and Drying Unit (Werner Mathis AG, Switzerland) in continuous operation, whereby cotton textile passes through spinach juice, is squeezed between two rollers and is then blown dry with 30°C air (thermostated). The swatches were aged for 3 weeks at 20°C, and were then kept at -18°C until use.

Swatches with mixed soiling were made by immersing cotton in the below mixture; squeezing between rollers; drying and aging for 2 days in air at 20°C.

```
Olive oil            14.4 weight %
Stearic acid          1.8 -       -
Monoglyceride         1.8 -       -
Gelatine              0.9 -       -
Deionized water      79.3 -       -
Carbon black          0.2 -       -
China clay            1.4 -       -
Indian ink            0.2 -       -
```

Washing tests were made in a Terg-O-Tometer (Jay C. Harris: Detergency Evaluation and Testing, Interscience Publishers Ltd. (1954), pp. 60-61) with 7 swatches (7 x 7 cm) and 700 ml detergent solution in each beaker. Conditions were 25°C, 10 min, 100 rpm. After rinsing and drying, reflectance (R) of the swatches at 460 nm was measured. The washing performance is expressed as delta R = R-$R_o$, where $R_o$ is the measurement without enzyme.

Protease of the invention (prepared as in Example 2) was compared to component I (prepared as in Example 2) and to Savinase® (an alkaline Bacillus protease, product of Novo Industri A/S, Denmark).

Results are given as $R_o$ and delta R versus protease type and dosage (in CPU/l). The below results are also shown in figures as indicated:

Example 3 (fig. 8)

| Det. I, spinach soiling | | | | | |
|---|---|---|---|---|---|
| | CPU/l | | | | |
| Protease | 0 $R_o$ | 0.025 delta R | 0.05 delta R | 0.075 delta R | 0.1 delta R |
| Invention | | 8.3 | 11.2 | 13.5 | 15.3 |
| Comp. I | 42.2 | 2.6 | 7.0 | 10.3 | 11.5 |
| Savinase | | 2.5 | 5.1 | 8.3 | 11.3 |

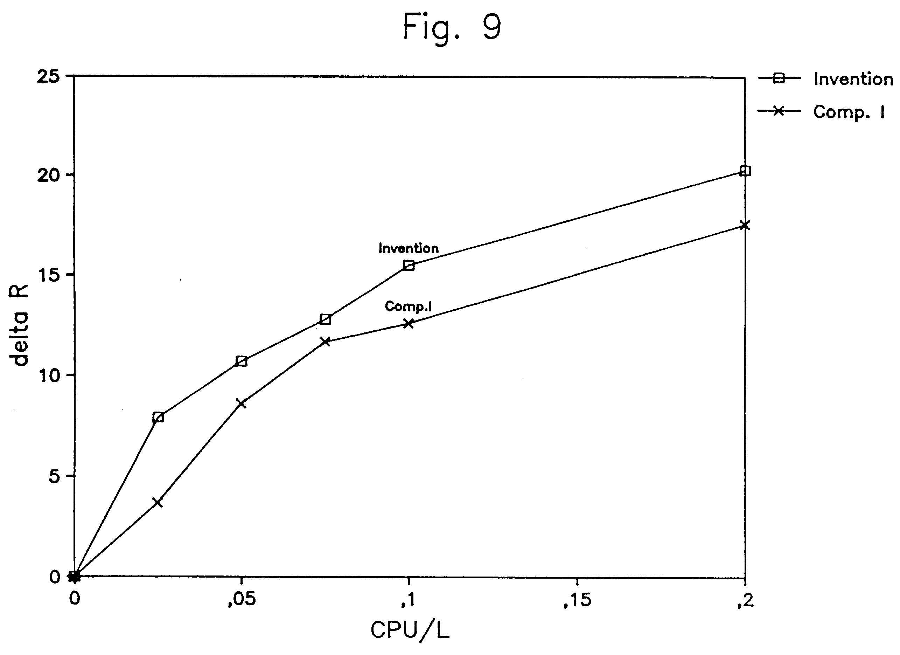

Example 4 (fig. 9)

| Det. II, spinach soiling | | | | | | |
|---|---|---|---|---|---|---|
| | CPU/l | | | | | |
| Protease | 0 $R_o$ | 0.025 delta R | 0.05 delta R | 0.075 delta R | 0.1 delta R | 0.2 delta R |
| Invention | | 7.9 | 10.7 | 12.8 | 15.5 | 20.3 |
| Comp. I | 43.4 | 3.7 | 8.6 | 11.7 | 12.6 | 17.6 |

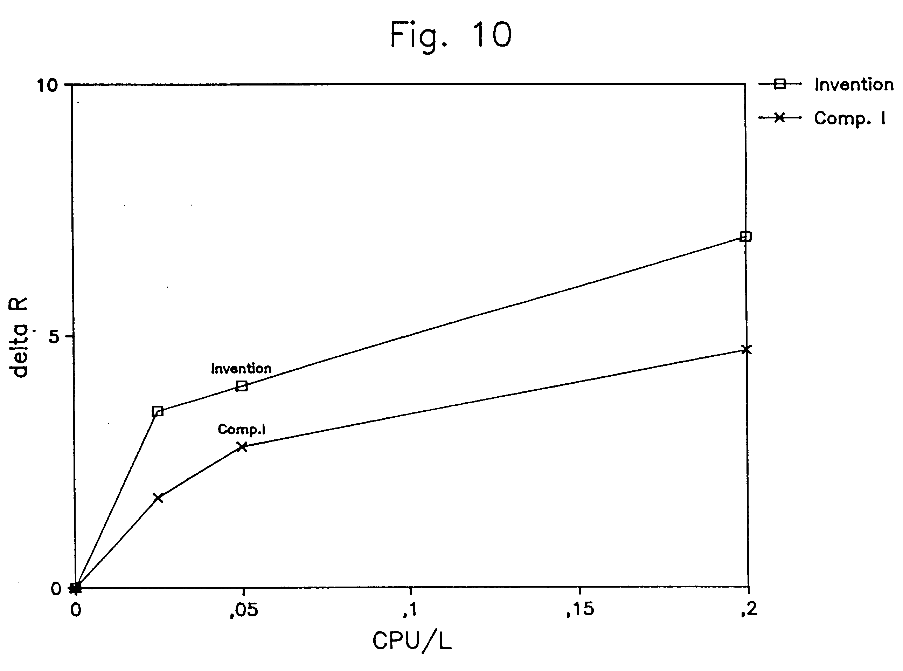

Example 5 (fig. 10)

| Det. IV, spinach soiling | | | | |
|---|---|---|---|---|
| | CPU/l | | | |
| Protease | 0 $R_o$ | 0.025 delta R | 0.05 delta R | 0.2 delta R |
| Invention | | 3.5 | 4.0 | 7.0 |
| Comp. I | 37.7 | 1.8 | 2.8 | 4.7 |

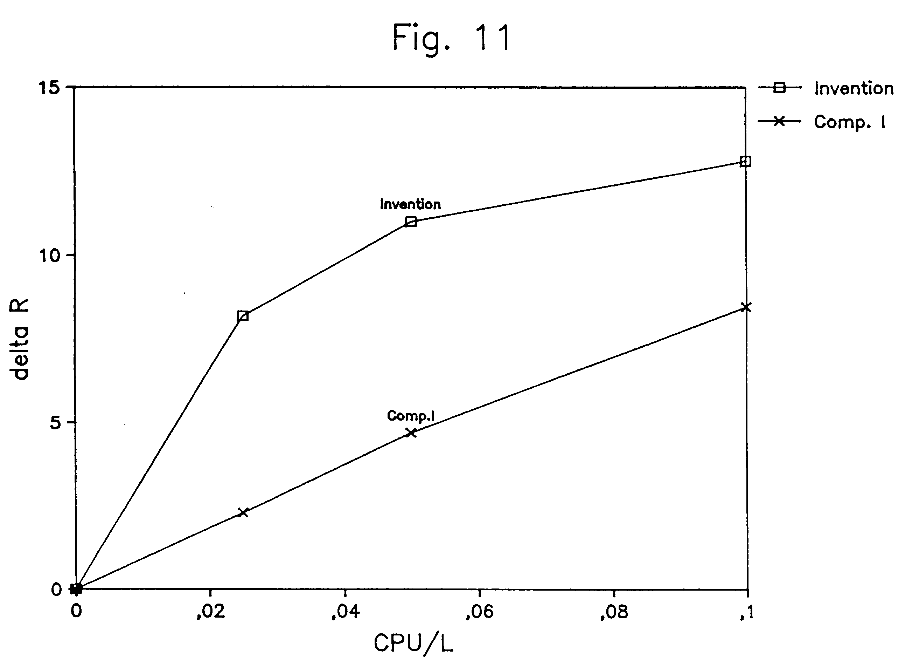

Example 6 (fig. 11)

| Det. I, mixed soiling | | | | |
|---|---|---|---|---|
| | CPU/l | | | |
| Protease | 0 $R_o$ | 0.025 delta R | 0.05 delta R | 0.1 delta R |
| Invention | | 8.2 | 11.0 | 12.8 |
| Comp. I | 21.0 | 2.3 | 4.7 | 8.5 |

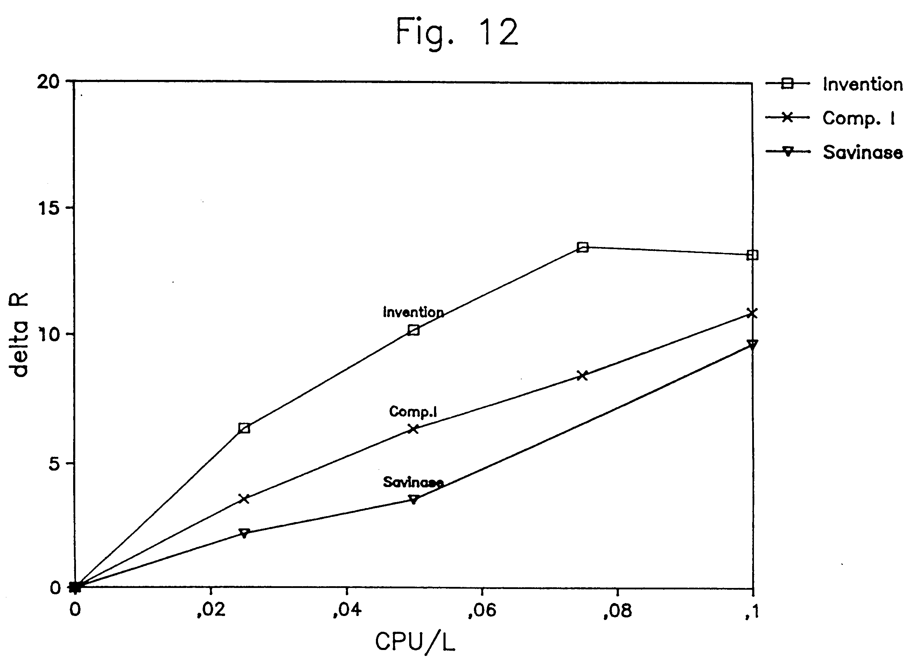

Example 7 (fig. 12)

| Det. III, spinach soiling | | | | | |
|---|---|---|---|---|---|
| | CPU/l | | | | |
| Protease | 0 $R_o$ | 0.025 delta R | 0.05 delta R | 0.075 delta R | 0.1 delta R |
| Invention | | 6.4 | 10.2 | 13.5 | 13.2 |
| Comp. I | 40.5 | 3.6 | 6.4 | 8.5 | 10.9 |
| Savinase | | 2.2 | 3.6 | | 9.7 |

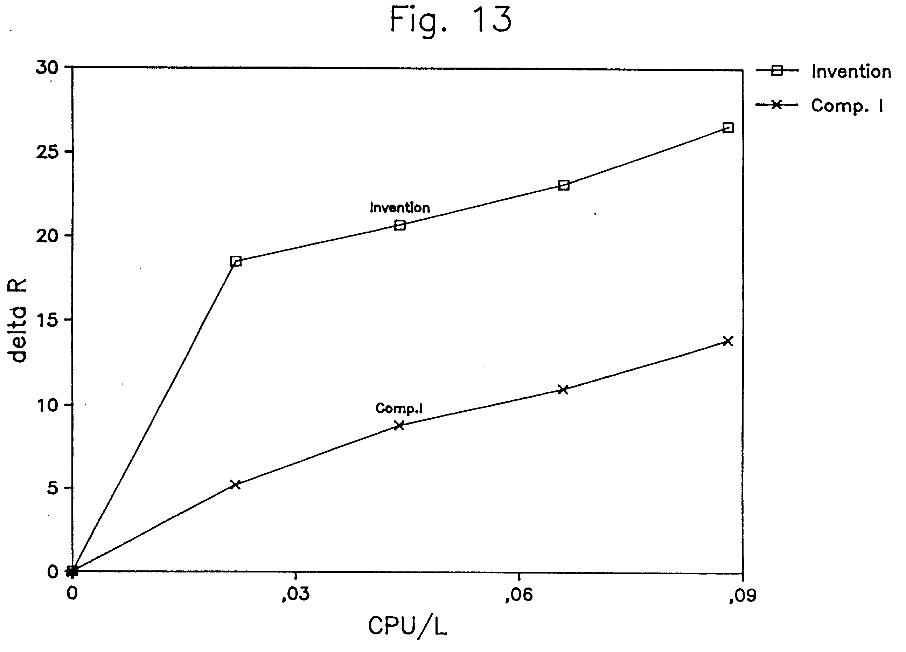

Example 8 (fig. 13)

| Det. II, mixed soiling | | | | | |
|---|---|---|---|---|---|
| | CPU/l | | | | |
| Protease | 0 $R_o$ | 0.022 delta R | 0.044 delta R | 0.066 delta R | 0.088 delta R |
| Invention | | 18.5 | 20.7 | 23.1 | 26.6 |
| Comp. I | 21.5 | 5.2 | 8.8 | 11.0 | 13.9 |

The detergent formulations tested cover pH 8-10, with various builders (phosphate and non-phosphate) with and without perborate, and with anionic and non-ionic surfactant. Thus, a wide range of typical formulations for liquid and powder detergents have been covered. At all these conditions, protease of the invention showed superior washing effect on the basis of CPU activity.

EP 0 471 265 B1

## EXAMPLE 9

Washing trials with protease mixtures

Detergent No. I of Examples 3-8 was used. Swatches and washing conditions were as in example 3-8. The protease of the invention, component I and various mixtures of these were added at total dosage up to 0.10 CPU/l.

| Protease ratio | CPU/l | | | |
|---|---|---|---|---|
| Invention : comp. I | $0 R_o$ | 0.01 delta R | 0.05 delta R | 0.10 delta R |
| 0 : 100 | 45.1 | 1.8 | 7.1 | 9.7 |
| 50 : 50 | 44.7 | 1.7 | 9.1 | 11.5 |
| 75 : 25 | 44.7 | 2.1 | 8.8 | 11.2 |
| 90 : 10 | 45.1 | 2.0 | 9.1 | 11.2 |
| 99 : 1 | 45.1 | 2.2 | 9.5 | 12.2 |
| 100 : 0 | 44.7 | 2.2 | 11.0 | 14.3 |

It is seen that increasing purity of the specific protease gives increased washing performance.

## Claims

1. A protease, characterized by:
   a) being a serine protease
   b) the ability to hydrolyze the oxidized B-chain of bovine insulin, so that a chromatogram shows only two major hydrolysis products
   c) showing immunochemical identity to a protease obtained by cultivation of Fusarium sp. DSM 2672
   d) an isoelectric point of about 9-10
   e) ability to hydrolyze Bz-Arg-pNA
   f) essentially no hydrolysis of Suc-AAPF-pNA
   g) essentially the same activity towards casein at pH 9 and pH 11

2. A protease preparation, in which the protease of Claim 1 makes up more than 90% of the total protease activity present.

3. A protease preparation according to Claim 2, which is essentially devoid of other proteases.

4. A protease preparation according to Claim 2 or 3 in the form of a non-dusting granulate, a stabilized liquid or a protected enzyme.

## Patentansprüche

1. Protease, gekennzeichnet durch:
   (a) das Merkmal, eine Serin-Protease zu sein
   (b) die Fähigkeit, die oxidierte B-Kette von Rinder-Insulin zu hydrolysieren, so daß ein Chromatogramm nur die beiden Haupthydrolyseprodukte zeigt
   (c) das Zeigen einer immunchemischen Identität zu einer Protease die durch die Kultivierung von Fusarium sp. DSM 2672 erhalten wird
   (d) einen isoelektrischen Punkt von ungefähr 9-10
   (e) die Fähigkeit, Bz-Arg-pNA zu hydrolysieren
   (f) im wesentlichen keine Hydrolyse von Suc-AAPF-pNA
   (g) im wesentlichen die gleiche Aktivität gegenüber Casein bei pH 9 und pH 11.

2. Proteasepräparat, in dem die Protease von Anspruch 1 mehr als 90% der gesamten vorhandenen Proteaseaktivität bildet.

3. Proteasepräparat nach Anspruch 2, das im wesentlichen ohne andere Proteasen ist.

10

**4.** Proteasepräparat nach Anspruch 2 oder 3 in der Form eines nichtstaubenden Granulats, einer stabilisierten Flüssigkeit oder eines geschützten Enzyms.

**Revendications**

**1.** Protéase caractérisée en ce qu'elle :
a) est une sérine protéase
b) est capable d'hydrolyser la chaîne B oxydée de l'insuline bovine d'une manière telle qu'un chromatogramme ne montre que deux produits principaux d'hydrolyse
c) présente la même identité immunochimique que celle d'une protéase préparée par culture de <u>Fusarium sp</u>. DSM 2672
d) possède un point isoélectrique d'environ 9 à 10
e) est capable d'hydrolyser Bz-Arg-pNA
f) ne provoque essentiellement pas d'hydrolyse de Suc-AAPF-pNA
g) présente essentiellement la même activité vis à vis de la caséine à pH 9 et pH 11.

**2.** Préparation de protéase où la protéase selon la revendication 1 représente plus de 90 % de l'activité protéasique totale présente.

**3.** Préparation de protéase selon la revendication 2, essentiellement dépourvue d'autres protéases.

**4.** Préparation de protéase selon la revendication 2 ou la revendication 3, sous la forme de granulés non-pulvérulents, de liquide stabilisé ou d'une enzyme protégée.

FIG. 1

Fig. 2

Fig.3

FIG. 4

FIG. 5

FIG. 6

2.56
3.86
4.70
7.53
10.13
11.03
12.10
13.00
13.66
14.76
16.96
17.56
18.16
18.63
19.26
19.90
21.13
22.43
23.13
24.63
25.73
26.63
28.13
29.56
30.26
32.10
33.03
33.96
36.93
45.90

FIG. 7

# Fig. 8

Fig. 9

Fig. 10

# Fig. 11

EP 0 471 265 B1

Fig. 12

Fig. 13